# EUROPEAN PATENT SPECIFICATION

(11) **EP 4 436 496 B1**
(45) Date of publication and mention of the grant of the patent: **31.12.2025**
(21) Application number: 22812726.2
(22) Date of filing: 16.11.2022
(51) Int. Cl.: A61B 17/14, A61B 17/15, A61F 2/46

(54) **A CUTTING ASSEMBLY FOR RESECTING A PATELLA**
SCHNEIDANORDNUNG ZUR RESEKTION EINER KNIESCHEIBE
ENSEMBLE DE COUPE POUR LA RÉSECTION D'UNE ROTULE

(30) Priority: 22.11.2021 GB 202116777
(43) Date of publication of application: 02.10.2024
(73) Proprietor: Eventum Orthopaedics Limited, Leeds, LS16 6QY (GB)
(72) Inventor: NAYBOUR, John, Leeds, LS16 6QY (GB); ATHERTON, Paul, Leeds, LS16 6QY (GB)
(74) Representative: EIP
(86) International application number: PCT/GB2022/052906
(87) International publication number: WO 2023/089310

(56) References cited:
- EP-A1- 2 540 233
- EP-A1- 2 540 234
- US-A- 5 147 365

## Description

### Background

This invention relates to a cutting assembly for resecting a patient's patella during knee surgery.

Some knee replacement procedures include replacement of the portion of the patella which acts against the femur during articulation of the joint with a patella implant component. Replacement involves removing the surface part of the patella which acts against the femur to prepare the patella to receive the patella implant component. The patella implant component has a bearing surface which is shaped to act against the femur, with a generally domed portion which facilitates sliding of the patella along the trochlear groove.

US-5129908 discloses a device which can be used to clamp the patella, and which can guide a reamer which is used to remove the surface part of the patella which acts against the femur. The document discloses a patella implant component which can be fitted to the prepared patella.

The device disclosed in US-5129908 is intended for clamping the native patella. The reamer is used to create a stepped surface including an annular surface formed by a set of outer cutters and a central raised surface formed by central cutters. The stepped surface matches the shape of the surface of the patella implant component which is fitted to the prepared patella.

WO-A-2008/112996 discloses a cutting block for use in preparing a patella during knee replacement surgery. The cutting block is hollow so that it can fit over the patella. Openings are formed in the walls of the cutting block in which the blade of a bone saw can be inserted, to engage the patella when located within the cutting block.

EP 2540233 A1 describes an orthopaedic surgical instrument assembly including a handle and a plurality of surgical tools configured to be selectively coupled with the handle. The handle includes a housing and a lever moveably coupled to the housing. Each surgical tool of the plurality of surgical tools includes a first clamping element and a second clamping element configured to move relative to the first clamping element. The second clamping element is configured to be coupled to the lever such that moving the lever relative to the housing advances the second clamping element toward the first clamping element to clamp a bone of a patient.

EP 2540234 A1 describes an orthopaedic surgical instrument including a patella resection guide. The patella resection guide includes a body having a substantially planar upper surface that defines a patella cutting guide surface and an inner wall defining an aperture through the body. The aperture is configured to receive a patient's patella and the inner wall includes a first jaw having a first set of teeth extending inwardly into the aperture. The patella resection guide includes a second jaw positioned opposite the first jaw. The second jaw is and movable relative to the first jaw. The second jaw includes a second set of teeth extending inwardly toward the first jaw. The first jaw includes a first tooth of the first set of teeth having a length greater than any other tooth of the first set of teeth, and the second jaw has a second tooth of the second set of teeth that has a length greater than any other tooth of the second set of teeth.

US 5147365 A describes a patella osteotomy guide comprising a plier-like appliance with curved jaws for grasping a patient's patella. A row of teeth faces inwardly from the jaws. The teeth are generally of pyramid shape, but a vertex of each tooth lies in a plane containing a bottom side of the respective jaw. Each of the jaws has an integral saw capture slot and the tips of the jaws are extended. A rotating, calibrated stylus measures the position of the patella with respect to the integral saw capture slots. The rotating stylus also functions as a pivot or fulcrum about which the jaws and handles of the osteotomy guide rotate. Handles for the osteotomy guide are offset from the plane of the jaws. The rotating stylus can be displaced up and down and then locked into a selected position using a draw bar and captured balls. A bowed retaining ring imparts a certain amount of pre-load to the stylus and draw bar assembly to hold the assembly in a selected position.

### Summary

The present invention provides a cutting assembly for resecting a patient's patella during knee surgery, in which a patella clamp provides a reference surface for engaging the posterior surface of the patella, and a support surface for the cutting instrument relative to the clamp, and in which the assembly includes a connector arrangement which can be adjusted, for example by use of different connector inserts, to vary the inclination of the support surface relative to the reference surface.

The invention therefore provides a cutting assembly for resecting a patient's patella during knee surgery, which comprises:
a. a cutting instrument having a cutting head for cutting the patella on a selected resection plane,
b. a clamp for a patella having opposing jaws which can be moved towards one another to clamp the patella between them, the clamp providing:
   i. a reference surface for engaging the posterior surface of the patella,
   ii. a support surface for the cutting instrument relative to the clamp,
in which the assembly includes a connector arrangement which provides a physical connection between the reference and support surfaces to define the inclination of the support surface relative to the reference surface, and which can be adjusted to vary the inclination of the support surface relative to the reference surface.

The invention enables the inclination of a cutting instrument relative to a patella which is held during a cutting step by means of a clamp to be varied so that the inclination (and optionally also the location) of the patella surface which is created using the cutting instrument can be varied. This can be achieved by adjusting the connector arrangements (for example by changing one or more connector inserts mounted on or relative to the clamp) to vary the spatial relationship of the reference surface relative to the support surface. The variation can involve the angle of the cut so that the cut might be deeper towards the medial edge of the patella than towards the lateral edge, or deeper towards the lateral edge of the patella than towards the medial edge, or deeper towards the superior edge of the patella than towards the inferior edge, or deeper towards the inferior side of the patella than towards the superior edge. The variation can involve a combination of medial/lateral and superior/inferior inclinations.

Optionally, the connector arrangement comprises a plurality of connector inserts which can be mounted individually or in one or more combinations on or relative to the clamp, providing a physical connection between the reference and support surfaces which defines the inclination of the support surface relative to the reference surface, and in which variation of the inclination of the support surface relative to the reference surface involves changing one or more of the connector inserts that are mounted on or relative to the clamp.

Optionally, the connector inserts have markings on them which are indicative of respective inclination angles of the support surface relative to the reference surface which are provided by the clamp when assembled with the selected one or more connector inserts. For example, the markings might include a numerical indication of the inclination angle of the support surface relative to the reference surface. When each of the support and reference surfaces is planar (which will often but not necessarily always be the case), the markings might include a numerical indication of the angle between the planes defined by the support and reference surfaces, for example in degrees.

Optionally, the difference between the angle between the support surface and the reference surface that is defined by the first connector insert and the angle between the support surface and the reference surface that is defined by the connector insert is at least about 1°, preferably at least about 1.5°. Optionally, the difference is not more than about 4°, preferably not more than about 3°. For example the difference can be about 2.5°.

Alternatively or in addition, the markings might include an indication of the orientation of the inclination angle of the cutting instrument, for example whether the depth of the cut in the patella is greater medially or laterally, or is greater superiorly or inferiorly.

Optionally, a connector insert can be double ended so that the difference between the angle between the support surface and the reference surface that is defined by a first end of the connector insert and zero is half of the difference between the angle between the support and reference surfaces that is defined by the first end of the connector insert and the angle between the support and references surfaces that is defined by the second end of the connector insert. For example one end of a connector insert might define an angle between the support and reference surfaces which is +2.5° and the other end of a connector insert might define an angle which is -2.5°, or one end of a connector insert might define an angle between the support and reference surfaces which is +1.25° and the other end of a connector insert might define an angle which is -1.25°.

The cutting assembly of the invention can be used in a method of knee replacement surgery which comprises:
a. performing a first resection on the patient's patella,
b. fitting to the resected patella a patella sensor component comprising:
   i. a backing plate having at least one sensor which can generate a signal corresponding to the compressive load applied to the backing plate, and
   ii. a first shim which can be fitted to the backing plate and which, when fitted to the backing plate, provides a bearing surface for articulation with a bearing surface on the patient's femur,
c. articulating the knee joint to obtain information from the sensor concerning tension in soft tissue which is connected to the patella as the patella articulates against the patient's existing femoral bearing surface,
d. fitting a femoral component to the patient's femur,
e. articulating the knee joint to obtain information from the sensor concerning tension in soft tissue which is connected to the patella as the patella articulates against the surface provided by the femoral component,
f. identifying the location and inclination of a second resection of the patella to receive a patella implant component whose thickness is greater than that of the patella sensor component, to provide a desired tension in soft tissue which is connected to the patella as the patella articulates against the femoral implant component in the completed replacement joint,
g. performing the second resection on the patient's patella, and
h. fitting the patella implant component to the patient's patella.

This method makes possible the selection of an appropriate soft tissue tension after surgery because the thickness of the patella sensor component is less than that of the patella implant component. This means that the amount of tissue that is removed from the patella in a first resection to allow the patella sensor component to be fitted against the patella is less than the total amount of tissue that might be removed from the patella after a second resection which then allows the patella implant component to be fitted. The patella fitting will accommodate changes in the position or inclination or both of the femoral bearing surface in the implanted knee prosthesis compared with the femoral bearing surface in the joint prior to surgery. Such changes can be detected using a patella sensor component which can be used to monitor the compressive forces which are imposed on the sensor component between the patella and the femoral bearing surface, resulting from soft tissue by which the patella is held in place and pressed against the femoral bearing surface.

The method provides the possibility of comparing the compressive forces at the patellofemoral interface between (a) the femoral bearing surface prior to surgery (which will be provided by the native femur in a primary knee replacement procedure, or the surface of a primary femoral component in a revision procedure), and (b) the femoral bearing surface provided by a trial femoral component or a femoral implant component as fitted to the femur during the surgical procedure. It can be desirable in order to optimise the surgical outcome to minimise the differences between the measured compressive forces. This can be done by appropriate selection of the depth of the resection of the patella: a deeper resection to remove relatively more patella tissue results in reduced compressive forces. Differences in the compressive forces in one or more regions of the patella can be reduced by varying the inclination of the patella resection, for example by increasing its depth medially more than laterally, or laterally more than medially, or superiorly more than inferiorly, or inferiorly more than superiorly, or combinations thereof. The invention enables forces in soft tissue connected to the patella to be adjusted by changing the effective depth of the patella, for example so as to accommodate changes in the anterior-posterior position of the femoral bearing surface.

The assembly of the invention can therefore be used with an assembly for use in knee surgery, which comprises:
a. a patella sensor component comprising:
   i. a backing plate which can be fitted to the patella and which has at least one sensor which can generate a signal corresponding to the compressive load applied to the backing plate,
   ii. at least first and second shims which provide a patella bearing surface for articulation against a surface on the patient's femur, and which can be positioned individually or in one or more combinations between the backing plate and the patient's patella to define first and second positions of the bearing surface relative to the patella,
b. a cutting instrument having a cutting head for cutting the patella on a selected resection plane,
c. a clamp for a patella having opposing jaws which can be moved towards one another to clamp the patella between them, the clamp providing:
   i. a reference surface for engaging the posterior surface of the patella,
   ii. a support surface for supporting the cutting head of the cutting instrument, thereby locating the cutting instrument relative to the clamp,
d. at least first and second locator inserts which can be mounted individually or in one or more combinations on or relative to the clamp, providing a physical connection between the reference and support surfaces which define first and second positions of the support surface relative to the reference surface, and thereby define first and second positions for the resection plane relative to posterior surface of the patella,
in which the increment between the first and second positions of the bearing surface on the bearing plate relative to the patella matches the increment between the first and second positions of the support surface relative to the reference surface.

The posterior surface of the patella which is engaged by the reference surface of the clamp will often be the native articulation surface of the patella which acts against the femur prior to surgery. However, the posterior surface might be a surface which has resulted from a previous resection step.

Optionally, the backing plate and the shims include cooperating engagement features by which shims can be fastened to the backing plate. The engagement features can comprise resiliently deformable fingers on the backing plate or the shims, each having at least one latch at its end. Such fingers might deform in a bending mode when the backing plate and a shim are pressed together, and then spring back towards the undeformed configuration once the backing plate and the shim are fully engaged, for example with the latch received in a detent or a groove or against some other surface.

The connector arrangement can define the distance between the support surface and the reference surface, and in which the distance between the support surface and the reference surface can be varied by adjusting the connector arrangement.

Optionally, the connector arrangement comprises a plurality of spacer inserts which can be mounted individually or in one or more combinations on or relative to the clamp, providing a physical connection between the reference and support surfaces, and in which varying the distance between the support surface and the reference surface involves changing one or more of the spacer inserts that are mounted on or relative to the clamp.

Optionally, the assembly includes a plurality of spacer inserts which can be mounted individually or in one or more combinations on or relative to the clamp, providing a physical connection between the reference and support surfaces so that the distance between the support surface and the reference surface can be varied by changing one or more of the spacer inserts.

The variation in the distance between the support and reference surfaces in the clamp allows the amount of bone that is removed using the cutting instrument to be varied by adjusting the connector arrangement (for example, by changing one or more spacer inserts). This variation in the depth of the cut (so that, for example, a deeper cut results in the removal of more bone from the patella) is an option together with the variation in the inclination of the cut as discussed above.

Optionally, each of the spacer inserts has a marking on it which is indicative of the distance between the support surface and the reference surface which are provided by the clamp when assembled with the one or more selected spacer inserts.

Optionally, the difference between the distance between the support surface and the reference surface that is defined by the first spacer insert and the distance between the support surface and the reference surface that is defined by the second spacer insert is at least about 0.5 mm, preferably at least about 0.8 mm. Optionally, the difference is not more than about 2 mm, preferably not more than about 1.5 mm. For example the difference can be about 1 mm.

Information can be obtained concerning the tension in the soft tissue which is connected to a patient's patella as the patella articulates against the patient's existing femoral bearing surface (which could be the bearing surface provided by the native femur in a primary knee replacement procedure, or a surface of a previously implanted femoral component in a revision procedure) using a patella sensor component (which has been fitted to the patella after an initial resection of the patella to remove a thickness of bone which corresponds approximately to the thickness of the patella sensor component). This can be compared with information that is obtained subsequently, after fitting a femoral trial component or possibly a femoral implant component to the femur, when the patella articulates against the surface provided by the femoral trial or implant component. The tension in the soft tissue can then be increased by changing to a thicker bearing plate in the trial patella component. The tension in the soft tissue can be reduced by changing to a thinner bearing plate in the trial patella component. The balance in the tension in soft tissue on different sides of the joint (for example between medial and lateral sides) can be varied by changing the inclination of the patella sensor component.

A surgeon can calculate the correct amount to resect from the patella in order to obtain a desired tension in soft tissue connected to the patella (for example the quadriceps muscle), for example to match the tension in the soft tissue prior to the operation, by changing the thickness and optionally also the inclination of the trial patella component by a known amount. The sensors can provide load signals to a data processing device which can store the load data. Information concerning the sensed load can be displayed on a display device such as a monitor. The effect of changing the thickness and/or the inclination of the trial patella component throughout the range of motion of the joint can be observed. This information can help the surgeon to assess the relative suitabilities of different surgical options. For example, it can help the surgeon to match the load applied to the trial patella component by selecting a bearing plate having an appropriate thickness and/or inclination. The amount of additional bone that should be resected from the patella in order to accommodate the patella implant component can be calculated once the bearing plate with an appropriate thickness and/or inclination has been identified.

It can be convenient to use a patella sensor component which makes use of a set of shims to vary its effective thickness and the angle between its principal faces, in combination with a matching set of inserts which can be used on the clamp to set the distance between the reference and support surfaces and the angle between them. The distance between the reference and support surfaces and the angle between them can be set to match the effective thickness and angle between the principal surfaces of the patella sensor component by appropriate selection of inserts.

Optionally, at least one of the support surface and the reference surface is provided by one of the spacer inserts when mounted on or in relation to the clamp. For example, when the reference surface is provided by a spacer insert, a patella can be positioned within the clamp by pressing the patella against the spacer insert reference surface.

Optionally, the reference surface (which can be provided on each of the spacer inserts, but might be provided by another element of the clamp) has a profile which matches at least approximately the profile of the posterior surface of the patella so that the reference surface sits stably on the patella. When the patella has been resected in an initial resection step (for example so that a patella sensor component can be fitted against it as described above), the surface of the patella which is engaged by the reference surface will be approximately planar. It will then be preferred that the reference surface is approximately planar.

The reference surface can have one or more openings formed in it, for example, to facilitate assembly of each spacer insert with one of the connector inserts or with the clamp.

Optionally, the clamp includes a mount and each of the connector inserts has a mount feature for engaging the mount to locate a selected connector insert on the clamp.

Optionally, the clamp includes a mount and each of the spacer inserts has a mount feature for engaging the mount to locate a selected spacer insert on the clamp.

For example, the mount can comprise a rod and each of a plurality of inserts (especially each of a plurality of connector inserts) can have a channel formed in it so that a selected insert can be located on the clamp by sliding the insert along the rod.

Optionally, the mount can include a latch feature which can be engaged by an insert (such as a connector insert) which has been slid fully along the rod so that further movement of the insert along the rod is inhibited. For example, the latch feature can be a protrusion on the rod or other mount which is engaged by the insert with a snap fit. This might be achieved for example by means of a pair of jaws on the insert which can be opened to fit over the protrusion. At least the jaws on the insert can be made at least in part from a resiliently deformable material to facilitate opening of the jaws to fit over the protrusion.

Optionally, each of a plurality of connector inserts and each of a plurality of spacer inserts has a mateable connector feature for connecting a selected connector insert and a selected spacer insert to one another. For example, each of the connector inserts can have a first connector feature and each of the spacer inserts can have a second connector feature, which are arranged so that a first connector feature engages with a second connector feature to connect a selected connector insert to a selected spacer insert. It will often be preferred that the connector features on at least some of the connector inserts are the same, and that the connector features on at least some of the spacer inserts are the same, so that any of the connector inserts can be connected to any of the spacer inserts.

Optionally, the clamp includes a mount and each of the connector inserts has a location feature which can engage the mount and for mounting a selected connector insert on the clamp, and each of the connector inserts and each of the spacer inserts has a mateable connector feature for connecting a selected spacer insert to a selected connector insert which is mounted on the clamp. The connector insert can be selected to provide a desired angular relationship between the reference and support surfaces, and the spacer insert can be selected to provide a desired spacing between the reference and support surfaces. An example of a technique for identifying a desired angular relationship and a desired spacing can involve using a patella sensor component to measure the variation in tension in soft tissue connected to the patella during flexion, especially with shims which can be mounted on the patella sensor component to vary the effective thickness of the sensor component and/or the angle between its principal surfaces.

Optionally, each of the support surface and the reference surface is approximately planar and the assembly includes a first spacer insert and a second spacer insert, and the angle between the planes defined by the support and reference surfaces when the first spacer insert is connected to a selected connector insert is the same as the angle between the planes defined by the support and reference surfaces when the second spacer insert is connected to the selected connector insert.

The support surface can be fixed relative to the clamp, for example so that the support surface has a unique position relative to a patella which is held within the clamp. The support surface might be provided by a surface of the clamp. An example of a support surface that is provided by a surface of the clamp could be a planar surface which is provided by an arm which extends adjacent to the patella when it is held within the clamp in the normal position. The arm might be a part of a frame which extends at least part way around the patella when held within the clamp. The support surface can be an open surface so that a cutting head is not restrained from moving away from the support surface. The support surface can be a closed surface so that movement of the cutting head to separate it from the support surface is restricted. This can be achieved when the support surface is provided by an internal surface of a slot.

The support surface might be provided by a support component which can move relative to a patella which is held within the clamp. For example a support component might be capable of being fixed to a clamp in a desired position and inclination. This can be advantageous when access to a patella to fasten a clamp to it is restricted, resulting in the clamp possibly being aligned incorrectly. Proper alignment of the support surface can then be achieved by movement of the support component so that the support surface is in a desired position and inclination.

Optionally, the cutting head is a saw blade. Optionally, the clamp has a slot formed in it which the saw blade can move in with a reciprocating action, and the support surface is provided within the slot.

The cutting instrument can be a reamer with a rotating cutting head which can be directed towards the patella to remove tissue and so create a resected surface. The support surface can be provided by a collar which defines a bore. The cutting head can be inserted through the bore to engage the patella until a flange on the reamer engages an edge of the collar to prevent further movement of the cutting head towards the patella. The use of such depth stop arrangements in conjunction with reamer cutting instruments is known.

The clamp jaws can engage the patella on its anterior and posterior faces. However, it will often be preferred that the clamp jaws engage the patella on opposed edge surfaces. For example, the clamp might engage the patella on medial and lateral edge surfaces. An example of a suitable instrument is the Premium Patella Saw Guide which is available from Enztech Limited of Christchurch, New Zealand. This instrument includes a stylus arm which extends over the posterior surface of the patella when the patella is engaged within the instrument. Inserts as provided in the assembly of the invention can be mounted on the stylus arm.

Optionally, each of the support surface and the reference surface is approximately planar. This will be appropriate when the surface of the patella which is exposed in the resection step is planar for fitting against it of a patella implant component having a planar patella engaging surface. This will frequently be the case.

Optionally, the assembly includes a first connector insert and a second connector insert, in which, when the first connector insert is mounted on or in relation to the clamp, the plane defined by the reference surface is approximately parallel to the plane defined by the support surface, and in which, when the second connector insert is mounted on or in relation to the clamp, the angle between the planes defined by the support and reference surfaces is greater than 0°. For example, the angle might be at least about 1°, preferably at least about 1.5°. Optionally, the angle is not more than about 4°, preferably not more than about 3°. For example the angle can be about 1.25° or about 2.5°.

Optionally, the assembly includes a neutral connector insert which can be mounted on or in relation to the clamp, and in which, when the neutral connector insert is mounted on or in relation to the clamp, the plane defined by the reference surface is approximately parallel to the plane defined by the support surface.

Optionally, the assembly includes:
a. a primary insert which can be mounted on or in relation to the clamp so that, when the primary insert is mounted on or in relation to the clamp, the plane defined by the reference surface is parallel to the plane defined by the support surface, and
b. a plurality of spacer inserts which can be mounted individually or in one or more combinations on or relative to the clamp, providing a physical connection between the reference and support surfaces which defines the distance between the support surface and the reference surface so that the distance between the support surface and the reference surface can be varied by changing one or more of the spacer inserts,
in which the distance between the reference and support surfaces when the primary insert is mounted on the clamp is greater than the greatest of the distances between the reference and support surfaces that are available when any of the spacer inserts are mounted on the clamp.

A primary insert can be used in an initial stage of preparing a patella in which the patella is resected by removing a posterior portion of the patella including the patella bearing surface. The resulting posterior planar surface can be used in a sensing step using a patella sensor component in which compressive forces on the sensor component between the patella and the femoral bearing surface, resulting from soft tissue by which the patella is held in place and pressed against the femoral bearing surface, can be monitored, and used to determine the depth and inclination of a second resection step. The location of the second resection plane is determined by use with the clamp of a selected connector insert and a selected spacer insert. Sensors in a sensor component can be used to measure the effect of the tension in soft tissue and the position and magnitude of the intersegmental loads on the patella surface through the passive flexion cycle.

As an example, the distance between the reference and support surfaces when the primary insert is mounted might be at least about 4 mm, especially at least about 5 mm. The distance might be not more than about 8 mm, especially not more than about 7 mm, for example about 6 mm. This enables the assembly to be used in a resection step which results in a piece of bone having a thickness of 6 mm being removed from the patella, allowing a sensor component having a thickness of 6 mm to be positioned in the joint without changing the tension of soft tissue connected to the patella.

The connector arrangement can be arranged to define distances between the reference and support surfaces which are up to about 5 mm, or up to about 4 mm, or up to about 3 mm. An assembly can ideally include spacer inserts which define at least two distances between 1 mm and 4 mm (including the limits of the range). By way of example, when the primary insert results in a piece of bone having a thickness of 6 mm being removed and the selected spacer insert results in a piece of bone having a thickness of 2 mm being removed, the total amount of bone removed will have a thickness of 8 mm. If the patella implant component that is then used has a thickness of 9 mm, this will have the result of increasing the tension in soft tissue attached to the patella, which can be advantageous when the result of implanting a femoral joint component is to move the femoral bearing surface posteriorly.

The spacer inserts or the connector inserts or both can be made from polymeric material. Examples of suitable polymeric materials include polyolefins such as polyethylene and polypropylene, polyamides and polyesters, optionally with fibre reinforcement. The use of polymeric materials has the advantage of low cost and ease of manufacture by injection or other moulding processes, or by additive layer manufacturing (3-d printing). In addition, inserts can be designed so that, when made from polymeric materials, they include flexible portions which can function as living hinges, especially so that they can flex resiliently, for example so that they can function as latches.

### Introduction to the drawings

The invention is described below by way of example with reference to the accompanying drawings, in which:
Figure 1 is an isometric view of a clamp and primary, connector and spacer inserts of a cutting assembly which can be used to resect a patient's patella during knee surgery.
Figure 2 is an isometric view of the clamp together with a primary insert, positioned in contact with the posterior bearing surface of the patella, prior to resection by means of a bone saw.
Figure 3 shows a sensor, a set of shims which can be fitted individually to the sensor.
Figure 4 is an isometric view from below of the sensor shown in Figure 3 together with a selected one of the shims.
Figure 5 is an enlarged isometric view showing a primary insert, and the sets of connector and spacer inserts shown in Figure 1.
Figure 6 is an isometric sectioned view of the spacer inserts shown in Figures 1 and 5.
Figure 7 is an isometric view showing a connector insert, with a spacer insert shown in phantom mounted on the connector insert.
Figure 8 is an isometric view showing the connector and spacer inserts shown in Figure 8, mounted on the clamp.
Figures 9a to 9c are medial views of a patient's knee, with a sensor component positioned between the patella and the femur, at three stages in a knee replacement procedure.
Figures 10a to 10c are graphs which show the variation in load that is sensed by lateral, superior, medial and inferior load sensors on a patella sensor component during flexion, in which the y-axis units reflect the electrical output from the load sensors which is an indication of sensed load.

### Detailed description

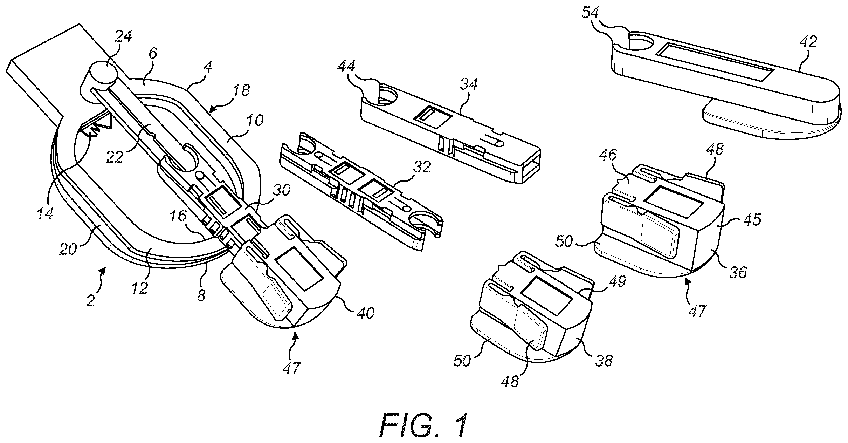

Referring to the drawings, Figure 1 shows a cutting assembly for resecting a patient's patella during knee surgery. The assembly can be used to locate the depth and inclination of the plane on which the patella is resected in order to prepare it for fitting of a patella implant component. The assembly is also used to support a cutting instrument which is used to perform the resection. As discussed below, the support surface in the assembly shown in Figure 1 is provided by a slot in a clamp for the patella and is suitable for supporting the blade of a bone saw as commonly used in orthopaedic surgery.

The assembly shown in Figure 1 has a clamp 2 having a frame 4 in the form of a planar hoop, with first and second ends 6, 8, and first and second sides 10, 12 which extend between the ends. The frame 2 has a first jaw 14 at the first end 10 of the frame and a second jaw 16 at the second end 12 of the frame. The first and second jaws 14, 16 point towards one another on opposite sides of the frame. The second jaw 16 is fixed to the frame at its second end 8. The first jaw 14 is located at the first end 6 of the frame and can slide towards and away from the second jaw 16. A patella can be gripped within the frame by sliding the first jaw 14 towards the second jaw 16 so that teeth on each of the jaws penetrate the tissue of the patella.

The frame has slots 18, 20 formed in it in the two sides 10, 12 which extend between the first and second ends 6, 8. The slots are coplanar so that a saw blade can extend through the first slot 18, across the frame, and through the second slot 20.

The clamp 2 has a stylus arm 22 which extends from the first end of the frame, parallel with the plane of the frame. The stylus arm is mounted on a pivot pin 24 so that it can be rotated about the pin. The stylus arm can be positioned against a patella when the clamp is positioned over a patella with the patella within the frame. The surface of the patella which is contacted by the stylus arm is usually the posterior surface, generally after eversion of the patella. The stylus arm is used as a point of reference for controlling the position of the patella within the frame of the clamp.

A connector arrangement in the assembly of the invention includes a set of connector inserts 30, 32, 34, a set of spacer inserts 36, 38, 40, and a primary insert 42. Each of the connector inserts has a bore within it whose cross-sectional shape is arranged so that it is a sliding fit on the stylus arm 22. One of the connector inserts 30 is shown in Figure 1 partially slid on to the stylus arm. Each of the connector inserts has a pair of flexible arms 44 adjacent to the open end of the bore. The arms can be opened so that they can fit around the pivot pin 24 at the end of the stylus arm 22. The connector inserts are formed from a polymeric material which allows the arms to be flexed resiliently so that they tend to restore to their unflexed state so as to engage the pivot pin and retain the connector insert in place on the stylus arm.

Each of the spacer inserts 36, 38, 40 has a hollow body 45 with a continuous top wall 46 and an open bottom face 47. The hollow body 45 can receive the end of one of the connector inserts 30, 32, 34, and has latch features including a pair of flexible latch fingers 48 on its side walls 49 by which any of the spacer inserts can be connected to any of the connector inserts. This is described in more detail below. The bottom face 47 of each of the spacer inserts 36, 38, 40 is provided by a pair of bone engaging flanges 50 which can be positioned against a patella, especially the posterior face of a patella which has been everted.

The connector inserts 30, 32, 34 differ from one another in terms of the angle between (a) the features by which they engage the stylus arm and (b) the features by which they are engaged by a selected one of the spacer inserts. The spacer inserts 36, 38, 40 differ from one another in terms of the distance between the bone engaging flanges and the features by which the spacer inserts engage a selected one of the connector inserts. A spacer insert is selected to provide an appropriate spacing between the plane defined by the slots 18, 20 in the frame 2 and the surface 47 of the spacer insert that is provided by the bone engaging flanges 50. This determines the depth of the portion of bone that is removed from a patella that is located within the clamp 2 between jaws 14, 16, in contact with the bone engaging flanges 50. The selection of an appropriate connector insert determines the inclination of the surface provided by the bone engaging flanges 50 relative to the plane that is defined by the slots 18, 20. This therefore determines the inclination of the plane on which the patella is resected.

The primary insert 42 has an elongate body with a bore within it whose cross-sectional shape is arranged so that it is a sliding fit on the stylus arm 22, similar to the connector inserts 30, 32, 34 as discussed above. The primary insert has a pair of flexible arms 54 adjacent to the open end of the bore which can fit around the pivot pin 24 at the end of the stylus arm to retain the primary insert in place on the stylus arm, in the same way as the arms 44 on the connector inserts 30, 32, 34.

The primary insert has a pair of bone engaging flanges 56 at the end which is opposite the end with the flexible arms 54, for positioning against a patella, especially the posterior face of a patella which has been everted.

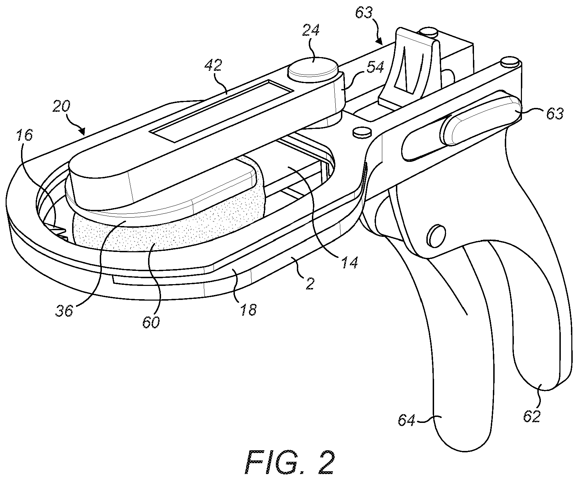

Figure 2 shows the clamp 2 with the primary insert 42 in place, slid fully on to the stylus arm 22 so that the flexible arms 54 are fitted around the pivot pin 24 and the primary insert is retained in place on the stylus arm.

A patella 60, which has been everted, is positioned within the frame 4 of the clamp 2, with its posterior surface against the bone engaging flanges 56 on the primary insert 42. The patella is held in place within the frame of the clamp by means of the jaws 14, 16.

The clamp which is shown in the drawings is sold under the trade mark Premium Patella Saw Guide by Enztech Limited of Christchurch, New Zealand. It has a fixed handle 62 and a pivoting handle 64. The pivoting handle is connected through a hidden ratchet linkage to the first jaw 14. The first jaw 14 can be moved towards the second jaw 16 against the action of hidden return springs by repeatedly squeezing the pivoting handle towards the fixed handle. The clamp has a pair of release buttons 63 which, when pressed together, release the first jaw so that it can retract under the action of the return springs, allowing the patella to be removed from the clamp. The distance between the plane defined by the slots 18, 20 in the frame 4 and the bone facing surface of the stylus arm 22 is 10 mm.

The plane defined by the slots 18, 20 is parallel to the plane that is defined by the bone engaging flanges 56 on the primary insert 42. The distance between the two planes is 4 mm. Consequently the depth of the portion of the patella that is removed posteriorly is about 6 mm. This is less than the depth of conventional patella implant components which frequently have a thickness of 8 mm, or 9 mm, or 9.5 mm, or 10 mm or more. Conventionally, the depth of the portion of the patella that is removed in a resection step corresponds to the thickness of the patella implant component that is to be fitted to the resected patella. The present invention involves an approach in which the patella which has been resected on the initial resection plane following use of the primary insert is assessed to identify the optimum location and inclination for the final resection plane on which the patella implant component is to be fitted. The location and inclination of the final resection plane can be optimised by varying the distance between initial and final resection planes, and the angle between them.

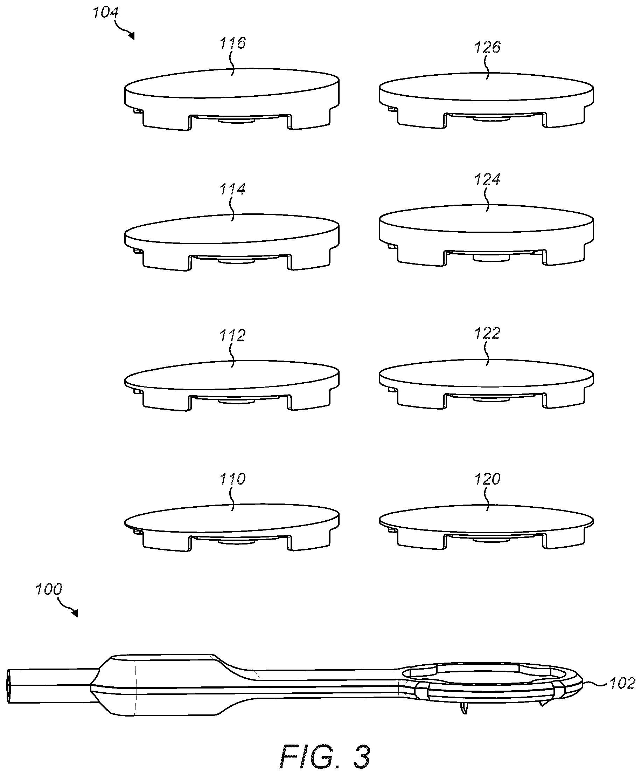

The resected patella following the initial resection step is assessed using a patella sensor component. Figure 3 shows a patella sensor component 100 which comprises a backing plate 102 with at least one sensor which can generate a signal corresponding to the compressive load applied to the backing plate. The sensor component includes a set of circular shims 104. Any of the shims can be fitted to the sensor backing plate and can provide a convex dome-shaped patella bearing surface for articulation against the anterior bearing surface on the patient's femur. The shims define different locations or inclinations or both of the patella bearing surface relative to the patella.

The shims are shown in Figure 3 in two sets of four. In the shims 110, 112, 114, 116 of the first set, the thickness of each shim is constant around the peripheral edge of its bearing surface. The thickness of the shim at the peripheral edge varies between individual shims of the first set, so that the thinnest shim 110 tapers towards zero at its peripheral edge. The thicknesses of the other three shims 112, 114, 116 at their peripheral edges are 1 mm, 2 mm and 3 mm respectively.

In the shims 120, 122, 124, 126 of the second set, the thickness of the shim varies around the peripheral edge of its bearing surface. The angle between the plane defined by the top of the peripheral edge and the plane defined by the bottom of the peripheral edge is greater than zero. In each of the shims 120, 122, 124, 126 of the second set shown in Figure 3, the angle is 2.5°. Another set of shims might be provided in which the angle between the plane defined by the top of the peripheral edge and the plane defined by the bottom of the peripheral edge has a different value, for example 1.25°.

In each of the shims 120, 122, 124, 126 of the second set, the thickness of the shim is greatest (as a consequence of its convex dome shape) at the centre of the shim. The thinnest shim 120 of the second set tapers towards zero at the point on its periphery where the thickness is at a minimum. The thicknesses of the other three shims 122, 124, 126 of the second set at the point on their peripheries where the thickness is at a minimum are 1 mm, 2 mm and 3 mm respectively.

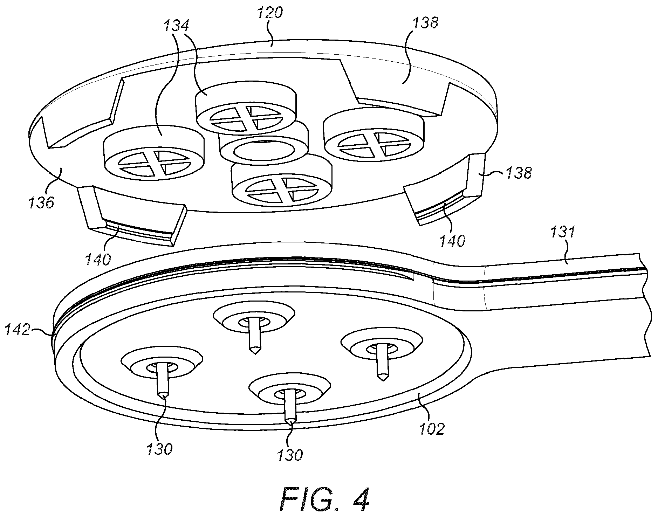

Figure 4 shows the backing plate 102 of a patella sensor component 100 together with the thinnest shim 120 of the second set of shims. The backing plate caries four sensors 130. The sensor component includes a handle 131 which carries signal wires from the sensors to a data processor.

Any suitable sensors can be used on the backing plate of the patella sensor component. For example, the sensor on the backing plate can comprise a flexible panel which is acted on by a protrusion on the bearing plate so that, when the patella sensor component is placed under a compressive load, the protrusion causes the panel to flex. Preferably, the panel should flex resiliently so that it returns to its unflexed position when a compressive load is removed. The panel can be connected with a strain gauge which can be used to measure the compressive load. It will often be preferred for the backing plate to have at least two flexible panels, especially at least three flexible panels, which are is acted on by respective protrusions on the bearing plate so that, when the patella sensor component is placed under a compressive load, the protrusions cause one or more of the panels to flex, so that the backing plate has at least two sensors or at least three sensors. When there is more than one flexible panel, each such panel can have a strain gauge associated with it to measure the compressive load applied to the patella sensor component. The patella sensor component might incorporate a Wheatstone bridge strain gauge array.

Additionally or alternatively, the backing plate might make use of force sensing resistors to measure a compressive load applied to the patella sensor component. For example, one or more force sensing resistors might be mounted on the backing plate to measure a compressive load applied to the patella sensor component (either integrally or as a separate sensor sheet). Suitable sensors can include piezoresistive force sensors. It is preferred that the sensors are thin and flexible so that they can be incorporated into the trial patella component. Sensors should be selected so that the range of forces that they can sense includes forces which might be sensed in the trial patella component. Preferably the output from the sensor varies linearly with the applied force, at least over the range of forces which might be sensed when the sensor is in use. An example of a force sensor which might be suitable for many applications is the FlexiForce HT201 sensor, available from Tekscan Inc.

Each of the sensors includes a pin 132 which is pressed against the surface of a patella when the sensor component is in use and ensures that each of the sensors is in physical contact with the patella, notwithstanding any surface irregularities in that surface.

Each of the shims 110, 112, 114, 116, 120, 122, 124, 126 has four protrusions 134 on the surface 136 which faces the backing plate. The protrusions are arranged so that they fit exactly over the sensors 130 on the backing plate. The shims have four deformable fingers 138 which depend from the surface 136 which faces the backing plate. Each of the fingers has an inwardly facing latch 140 at its end.

A selected shim such as the shim 120 shown in Figure 4 can be positioned over the backing plate 102 so that the protrusions 134 are positioned over the sensors 130 on the backing plate. Pressure applied to the shim to force it on to the backing plate causes the fingers 138 to deform outwardly over the edge of the backing plate. Continued applied pressure causes the shim to move towards the backing plate until the latches 140 on the fingers 138 spring inwardly into a peripheral groove 142 on the backing plate. Reception of the latches 140 in the groove 142 retains the shim on the backing plate. The shim can be released from the backing plate by application of a lifting force to the shim relative to the backing plate.

The orientation of the shim on the backing plate can be varied by rotating the shim successively through angles of 90°. This is advantageous when the shim is from the second set and therefore has a thickness which varies around the peripheral edge of its bearing surface. Rotation of the shim changes the direction of the inclination of the bearing surface, for example from M-L to A-P to L-M to P-A. Intermediate directions are possible by rotating the sensor (backing plate and shim assembly) through an angle of ±45°.

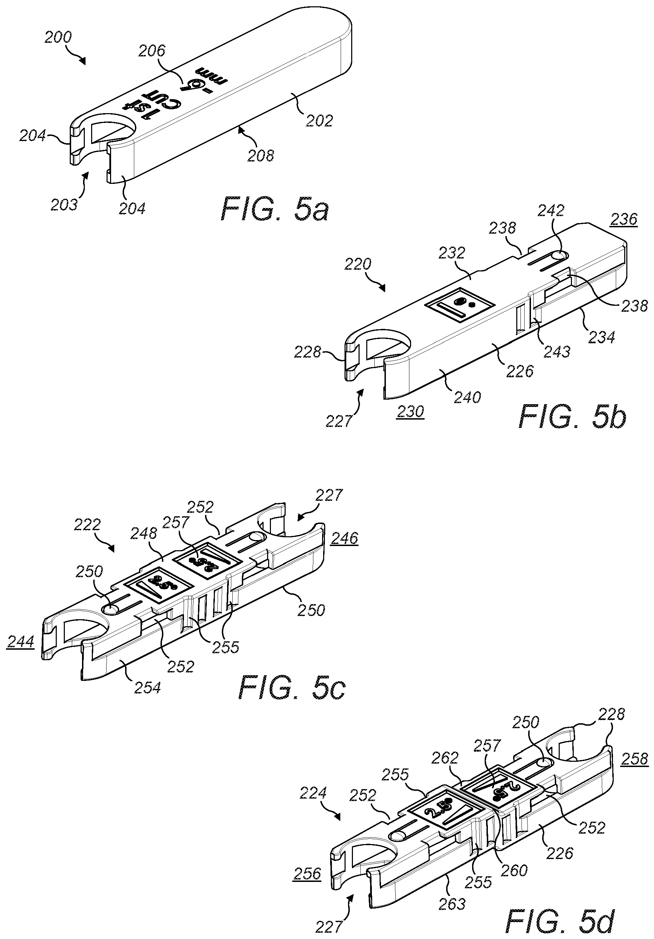

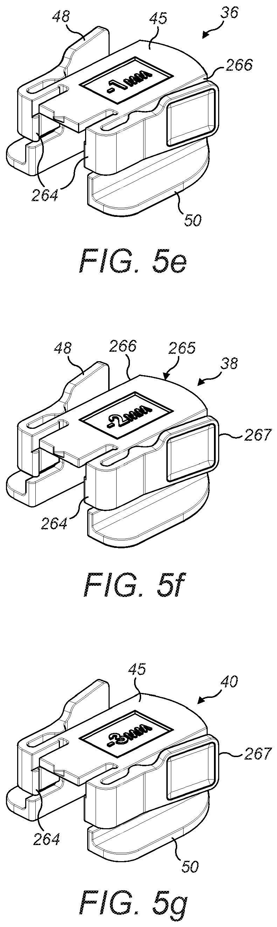

Figure 5a shows a primary insert 200 which has an elongate body 202 with a bore 203 within it whose cross-sectional shape is arranged so that it is a sliding fit on the stylus arm 22. The primary insert has a pair of flexible arms 204 adjacent to the open end of the bore which can fit around the pivot pin 24 at the end of the stylus arm to retain the primary insert in place on the stylus arm.

The primary insert 200 shown in Figure 5a has a constant cross-section along its length. The primary insert has markings 206 on its upper surface which identify the insert, to distinguish it from other inserts. The lower surface 208 of the primary insert 200 provides the reference surface, for positioning against a patella, especially the posterior face of a patella which has been everted. Unlike the primary insert shown in Figure 1, the primary insert shown in Figure 5a does not have bone engaging flanges on the reference surface at the end which is opposite the end with the flexible arms 204.

The distance between the plane defined by the slots 18, 20 of the frame 4 and the plane defined by the reference surface 208 on the primary insert 200 is 4 mm. This means that, if the arrangement of the stylus arm 22 and the slots 18, 20 is such that, in the absence of an insert on the stylus arm (so that the stylus can contact a patella positioned within the frame), the thickness of the patella tissue that is removed from the patella is 10 mm, the thickness of the patella tissue that is removed when using the primary insert 200 is 6 mm.

Figure 5b shows a neutral connector insert 220. Figure 5c shows an A-P connector insert 222. Figure 5d shows an M-L connector insert 224. Each of the connector inserts 220, 222, 224 has an elongate body 226 with a bore 227 within it whose cross-sectional shape is arranged so that it is a sliding fit on the stylus arm 22 and flexible arms 228 adjacent to the open end of the bore which can fit around the pivot pin 24 at the end of the stylus arm to retain the insert in place on the stylus arm.

The neutral connector insert 220 shown in Figure 5b is open and has flexible arms 228 at one end 230, and continuous upper and lower surfaces 232, 234 extending along the length of the insert. The upper and lower surfaces are essentially planar, and the planes defined by the upper and lower surfaces are perpendicular to the axis defined by the flexible arms, and the pivot pin when the connector insert is in place on the stylus arm. The bore 227 in the connector insert is closed at its other end 236 of the insert. L-shaped grooves 238 are provided in the side walls 240 of the insert towards the closed end 236. The grooves are open on the upper surface 232 of insert. The limb of the L-shaped groove which is open on the upper surface of insert is wider and shorter than the other limb of the groove. The insert has a spring finger 242 in its upper surface adjacent to the L-shaped grooves 238 and a recess 243 in each side wall adjacent to the closed end of each of the L-shaped grooves.

Figure 5c shows an A-P connector insert 222 which is open and has flexible arms 228 at each of its ends 244, 246. It has continuous upper and lower surfaces 248, 250 extending along the length of the insert. The upper and lower surfaces are essentially planar, and the angle between the planes defined by the upper and lower surfaces and a plane which is perpendicular to the axis defined by the flexible arms, and the pivot pin 24 when the connector insert is in place on the stylus arm, is 2.5°. This effectively results in the upper surface 248 of the A-P connector insert being inclined relative to the upper and lower surfaces of the stylus arm 22 when the arm is viewed along its length. The direction of the inclination (right-to-left or left-to-right when the stylus arm is viewed along its length) can be changed by turning the connector insert round through 180°.

The A-P connector insert 222 has two pairs of L-shaped grooves 252 in its side walls 254. The grooves are open on the upper surface 248 of the insert. The insert has spring fingers 250 in its upper surface adjacent to the L-shaped grooves 252 and a recess 255 in each side wall adjacent to the closed end of each of the L-shaped grooves.

Figure 5d shows an M-L connector insert 224 which is open and has flexible arms 228 at each of its ends 256, 258. A transverse step 260 is formed in each of the upper and lower surfaces 262, 263 of the M-L connector insert. The upper and lower surfaces are essentially planar on each side of the step, and the angle between the planes defined by the upper and lower surfaces and a plane which is perpendicular to the axis defined by the flexible arms, and the pivot pin 24 when the connector insert is in place on the stylus arm, is 2.5°. This effectively results in the upper surface 262 of the M-L connector insert being inclined relative to the upper and lower surfaces of the stylus arm 22 when the arm is viewed from one side. The direction of the inclination (right-to-left or left-to-right when the stylus arm is viewed from one side) can be changed by turning the connector insert round through 180°.

Similar to the A-P connector insert 222, the M-L connector insert has two pairs of L-shaped grooves 252 in its side walls 254. The grooves are open on the upper surface 262 of insert. The insert has spring fingers 250 in its upper surface adjacent to each of the L-shaped grooves 252 and a recess 255 in each side wall adjacent to the closed end of each of the L-shaped grooves.

Each of the connector inserts 220, 222, 224 has markings 257 on its upper surface which identify the insert, to distinguish it from other inserts. The markings provide the user with information concerning the inclination of the upper surface of each insert relative to a plane which is perpendicular to the axis defined by the pivot pin 24 when the insert is in place on the stylus arm.

While the A-P and M-L connector inserts which are shown in Figures 5c and 5d are double ended (with flexible arms for engaging the clamp pivot pin, and grooves and a flexible finger for engaging a spacer insert, at both ends), it might be preferred for some situations to provide single ended connector inserts whose appearance will be similar to that of the neutral connector insert shown in Figure 5b. A set of connector inserts will then include two A-P connector inserts in which the inclination angles are +2.5° and -2.5° (or some other value such as 1.25°) respectively, and two M-L connector inserts in which the inclination angles are +2.5° and -2.5° (or some other value such as 1.25°) respectively.

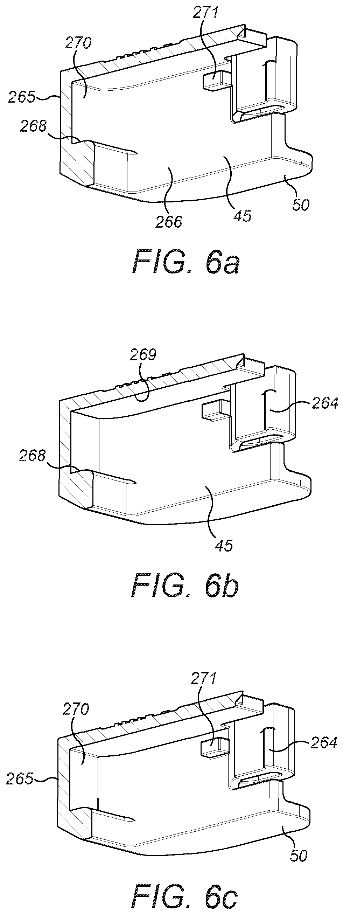

Figures 5e to 5g and Figures 6a to 6c show the spacer inserts 36, 38, 40 which are shown in Figure 1. Each of them has a hollow body 45 which can be fitted on to a free end of a selected one of the connector inserts 220, 222, 224. Figures 6a to 6c are sectioned isometric views, viewed from below, showing details of the spacer inserts within their hollow bodies.

Each of the spacer inserts 36, 38, 40 has a pair of bone engaging flanges 50 for positioning against a patella, especially the posterior face of a patella which has been everted, and an end wall 265 and a pair of side walls 266. Just one of the side walls can be seen in each of Figures 6a to 6c. The latch fingers 48 provided on each side wall have an inwardly facing detent 264 at its end. Each of the latch fingers 48 includes a lever arm 267 which can be pressed towards the body of the insert.

Each of the spacer inserts 36, 38, 40 has a step 268 in its closed end wall 265 which defines a pocket 270 between the step and the underside of the top wall 269 of the insert.

A locator flange 271 is provided on the inside of the side wall 266 of each spacer insert, adjacent to the latch finger 48. The length of the flange (measured along the axis which extends between the open and closed ends of the insert) is slightly less than the width of the short-open ended limb of the L-shaped groove in each of the connector inserts. The thickness of the flange (measured along the axis which extends between the continuous top wall 46 and the open bottom face 47 of the spacer insert) is slightly less than the width of the long-closed ended limb of the L-shaped groove in each of the connector insert. This arrangement means that a spacer insert can be positioned on the top surface of a connector insert with the locator flanges aligned with the open ends of the L-shaped grooves in the side walls of the connector inserts. The spacer insert can drop on to the connector insert until the locator flange reaches the end of the short limb of the L-shaped groove. The spacer insert can then be slid along the connector insert so that the locator flange slides towards the closed end of the long limb of the L-shaped groove, until the end of the stylus arm 22 is received in the pocket 270 between the step 268 and the underside of the top wall of the insert, and the detent 264 on the latch finger is received in the recess in the side wall of the connector insert. The engagement of the locator flange in the long limb of the L-shaped groove, and the engagement of the end of the stylus arm 22 in the pocket 270, accurately fix the spacer arm against up and down movement relative to the stylus arm.

The spring finger 242 in the top surface of each of the connector inserts presses against the underside of the top wall 269 of the selected spacer insert.

The spacer inserts 36, 38, 40 shown in Figures 5e to 5g and 6a to 6c differ from one another in terms of the distance between the top wall 269 of the hollow body 45 and the bone engaging flanges 50. The distance is greatest in the spacer insert shown in Figures 5e and 6a and least in the spacer insert shown in Figures 5g and 6c. Each of the spacer inserts has markings 272 on its top wall which is indicative of the distance between the support surface provided by the slots 18, 20 in the clamp and the bone engaging surface provided by the bone engaging flanges 50.
- with the spacer insert 36 mounted on a connector insert and the spacer arm 22, the distance between the plane defined by the slots 18, 20 of the frame 4 and the plane defined by the bone engaging surface provided by the bone engaging flange 50 is 9 mm. This means that, if the arrangement of the stylus arm 22 and the slots 18, 20 is such that, in the absence of an insert on the stylus arm (so that the stylus can contact a patella positioned within the frame), the thickness of the patella tissue that is removed from the patella is 10 mm, the thickness of the patella tissue that is removed when using the spacer insert 36 is 1 mm, making a total resection of 7 mm when the resection using the primary insert is 6 mm.
- with the spacer insert 40 mounted on a connector insert and the spacer arm 22, the distance between the plane defined by the slots 18, 20 of the frame 4 and the plane defined by the bone engaging surface provided by the bone engaging flange 50 is 7 mm. This means that, if the arrangement of the stylus arm 22 and the slots 18, 20 is such that, in the absence of an insert on the stylus arm (so that the stylus can contact a patella positioned within the frame), the thickness of the patella tissue that is removed from the patella is 10 mm, the thickness of the patella tissue that is removed when using the spacer insert 40 is 3 mm, making a total resection of 9 mm when the resection using the primary insert is 6 mm.

The amount of bone removed using different combinations of the spacers shown in the drawings is as follows:

| Shim | Insert which contacts patella | Depth of resection | Total depth of removed bone | Relative location of patella implant posterior surface |
|---|---|---|---|---|
| 0 mm (110) | Primary insert (42) | 6 mm | 6 + 0 = 6 mm | +3 mm |
| 1 mm (112) | Spacer insert (36) | 1 mm | 6 + 1 = 7 mm | +2 mm |
| 2 mm (114) | Spacer insert (38) | 2 mm | 6 + 2 = 8 mm | +1 mm |
| 3 mm (116) | Spacer insert (40) | 3 mm | 6+3= 9 mm | 0 |

The first column in the table indicates the shims that might are used with the backing plate to identify the optimum soft tissue tension across the knee joint. This informs the surgeon as to which of the spacer inserts should be used in a second resection step (if that is required). In the example set out in the table, the shim is one whose thickness is constant around its peripheral edge (110, 112, 114, 116). The connector insert to which a selected one of the spacer inserts is mounted is the neutral connector insert 220.

In another example, the shim might be one whose thickness varies around its edge (12, 122, 124, 126), with an inclination angle between the plane defined by the top of the peripheral edge and the plane defined by the bottom of the peripheral edge is greater than zero, for example 1.25° or 2.5°. In this case, the connector insert will be one which be an A-P connector insert or an M-L connector insert, for example as shown in Figures 5c and 5d.

The second column in the table identifies the insert which is provided on the stylus arm and which provides the surface for contacting the patella to locate the patella in the clamp during the resection step. It is the primary insert 42 in the initial cut which removes 6 mm of bone from the posterior of the patella, including the native patella bearing surface. It is one of the spacer inserts 36, 38, 40 in a second cut (if required) which removes an additional 1 mm, 2 mm or 3 mm layer of bone, as noted in the third column.

The last column in the table indicates the location of the posterior surface of a patella implant which is fitted to the resected patella, relative to the posterior surface of the patella prior to the procedure. In this example, the thickness of the patella implant that is used is 9 mm. By way of explanation, when the spacer insert 38 is used in the second resection step, the position of the posterior surface of the patella implant is 1 mm posterior of the native patella posterior surface. This can be appropriate to increase tension in soft tissue attached to the patella (such as the quadriceps muscle), for example because the anterior facing portion of the femoral bearing surface is located posteriorly of the native femoral bearing surface.

The orientation of the inclination of the plane of the second resection can be selected by appropriate selection of an appropriate connector insert, so that the second resection plan is inclined on the A-P axis or on the M-L axis. The orientation can be fine tuned by rotating the clamp relative to the patella (before the patella is gripped between the jaws 14, 16) through an angle of ±45°.

A spacer insert which is fitted to the A-P connector insert 222 or the M-L connector insert 224 masks one of the sets of markings on the connector insert. This means that, once the spacer insert has been fitted to the connector insert, a surgeon is able to read from the assembled inserts the information concerning the inclination of the resection plane in the patella from the markings on the connector insert that continue to be visible, and the depth of the resection from the markings on the spacer insert.

A spacer insert can be released from a connector insert to which it is connected by squeezing the latch fingers 48 towards one another and towards their respective side walls. By a lever action, this causes the detents 264 on the latch fingers to release from the recesses in the side walls of the connector insert so that the spacer insert can be slid along the connector insert. The locator flange thereby slides along the long limb of the L-shaped slot. The spacer insert can then be lifted off the connector insert, with the locator flange sliding along the short limb of the L-shaped slot.

A connector insert can be released from engagement with the pivot pin 24 by pulling the insert along the stylus arm 22, causing the flexible arms on the connector insert to splay.

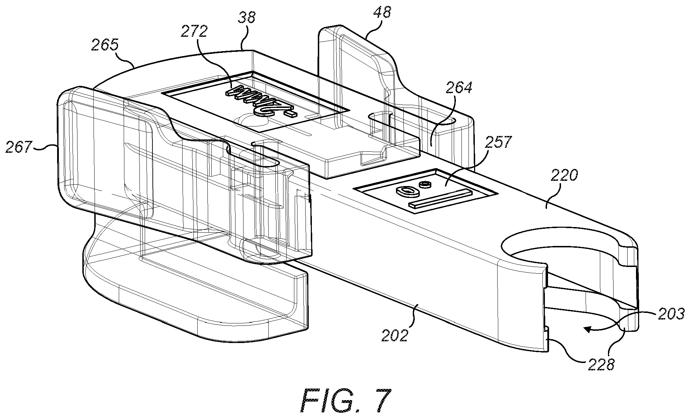

Figure 7 shows the neutral angle connector insert 220 and the spacer insert 38, with the spacer insert fully engaged with the connector insert, as described above.

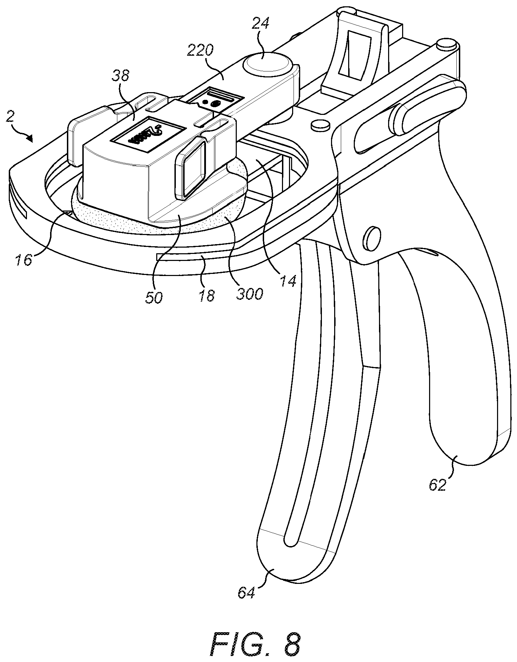

Figure 8 shows the neutral angle connector insert 220 and the spacer insert 38 assembled on the stylus arm 22 of the clamp 2, with an everted patella 300 held within the clamp by means of the jaws 14, 16, and the bone engaging flange 50 on the spacer insert 38 engaging the posterior surface of the patella.

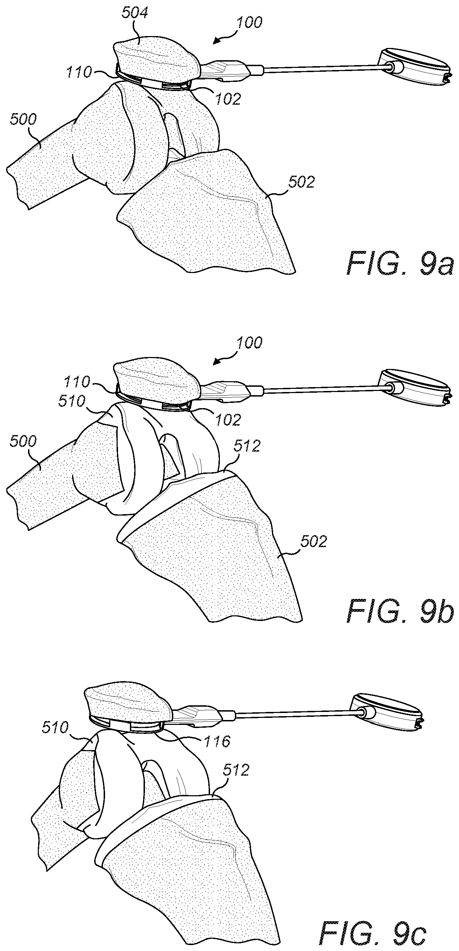

Figure 9a shows a patient's knee joint schematically, showing the femur 500 and the tibia 502 with the native femoral and tibial bearing surfaces intact. The patella 504 has been resected to a depth of 6 mm using the primary insert 42 on the clamp, as described above.

The patella sensor component 100 is positioned against the posterior resected surface of the patella. The sensor component has a shim 110 clipped to it. The shim is the thinnest of the shims of the first set, having a constant thickness around its peripheral edge. The thickness of the patella sensor component as used at this stage in the procedure is 6 mm, and is therefore the same as the thickness of the patella tissue that is removed in the initial resection step. The joint can be flexed with the sensor component in place on the posterior face of the patella and information about the compressive forces to which the sensor component is subjected can be obtained from the sensors 130 on the backing plate of the sensor component which can be seen in Figure 4.

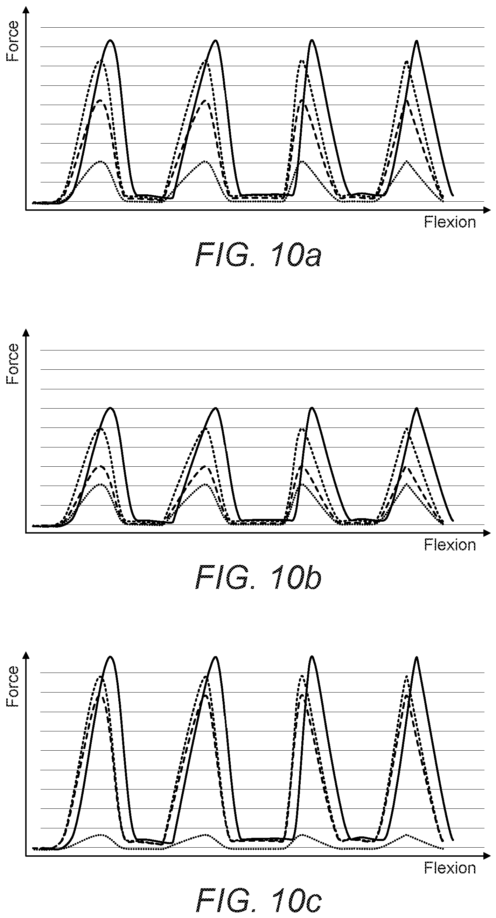

Figure 10a is a graph which shows how the compressive force that is measured using each of the sensors 130 varies during articulation of the joint.

Figure 9b shows the patient's knee joint after preparation of the femur and the tibia and fitting of a femoral trial component 510 and a tibial trial component 512. The surgical steps to prepare the femur and the tibia are well known.

Figure 9b shows the patella sensor component 100 positioned against the posterior resected surface of the patella, with the same shim 110 clipped to the sensor component as in the assessment of the native joint as shown in Figure 9a. Flexing the joint provides information about changes in the compressive forces to which the patella sensor component is subjected as a result of the surgical steps to fit the femoral and tibial trial components.

Changes to the forces to which the patella sensor component is subjected give rise to corresponding changes in the soft tissue by which the patella is held in place, notably the quadriceps muscle. The present invention enables such changes to be identified and to be minimised.

Figure 10b is a graph showing how the compressive force that is measured using the sensors 130 varies during articulation of the joint when the femoral and tibial trial components 510, 512 are in place. In this example, it can be seen that the compressive forces are reduced compared with those measured in the native joint. This might be for example because the anterior bearing surface provided by the femoral trial component is located posteriorly relative to the bearing surface provided by the native femur. A consequence is that the tension in soft tissue attached to the patella is reduced. This can compromise the surgical outcome, for example with the possibility of patellofemoral pain.

Figure 9c shows the patient's knee joint with the femoral and tibial trial components 510, 512 in place, and with a shim 116 attached to the backing plate 102 of the patella sensor component in the place of the shim 110 shown in Figures 9a and 9b. As discussed above, the shim 116 which is shown in Figure 9c is thicker than the shim 110 which is shown in Figures 9a and 9b. This results in the patella being located more anteriorly relative to the femur compared with its position in the arrangements shown in Figure 9b, so that the compressive forces to which the patella sensor component is subjected, and the tension in the soft tissue attached to the patella, are increased. The compressive forces to which the sensor component is subjected are depicted in the graph in Figure 10c.

Use of the components provided by the invention can involve identifying the shim (which might be any of the shims shown in Figure 3) which, when attached the backing plate as shown in Figure 9c, gives rise to variations in the compressive forces which most closely match the variations in the native knee as shown in Figure 10a. This enables the surgeon to select connector and spacer inserts for use with the clamp to perform a second resection. The inserts are selected by an analysis such as that described above with reference to the data in the table.

Patella implant components are frequently rotationally symmetrical. This can mean that the surgeon does not have to consider the rotational orientation of a patella implant component when positioned on a resected patella. Some patella implant components might not be rotationally symmetrical. For example, the apex of a domed patella implant component might be located closer to one edge than to an opposite edge. The assembly of the invention can be used to identify an optimum rotational orientation for such an implant component, in which the forces to which it is subjected, and which are applied to the surface of a femoral implant component with which the patella implant component articulates, are appropriately balanced.

## Claims

1. A cutting assembly for resecting a patient's patella (504) during knee surgery, which comprises:
a. a cutting instrument having a cutting head for cutting the patella on a selected resection plane,
b. a clamp (2) for a patella having opposing jaws (14, 16) which can be moved towards one another to clamp the patella between them, the clamp providing:
i. a reference surface (208) for engaging the posterior surface of the patella,
ii. a support surface for supporting the cutting head of the cutting instrument, thereby locating the cutting instrument relative to the clamp,
in which the assembly includes a connector arrangement which provides a physical connection between the reference and support surfaces to define the inclination of the support surface relative to the reference surface,
**characterised in that** the connector arrangement can be adjusted to vary the inclination of the support surface relative to the reference surface.

2. A cutting assembly as claimed in claim 1, in which the connector arrangement comprises a plurality of connector inserts (30, 32, 34, 220, 222, 224) which can be mounted individually or in one or more combinations on or relative to the clamp, providing a physical connection between the reference and support surfaces which defines the inclination of the support surface relative to the reference surface, and in which variation of the inclination of the support surface relative to the reference surface involves changing one or more of the connector inserts that are mounted on or relative to the clamp, preferably in which the connector inserts have markings (257)_on them which are indicative of respective inclinations of the support surface relative to the reference surface.

3. A cutting assembly as claimed in claim 1 or claim 2, in which the connector arrangement defines the distance between the support surface and the reference surface, and in which the distance between the support surface and the reference surface can be varied by adjusting the connector arrangement.

4. A cutting assembly as claimed in claim 3, in which the connector arrangement comprises a plurality of spacer inserts (36, 38, 40) which can be mounted individually or in one or more combinations on or relative to the clamp, providing a physical connection between the reference and support surfaces, and in which varying the distance between the support surface and the reference surface involves changing one or more of the spacer inserts that are mounted on or relative to the clamp, preferably in which the spacer inserts have markings (272) on them which are indicative of distances between the support surface and the reference surface.

5. A cutting assembly as claimed in claim 4, in which at least one of the support surface and the reference surface is provided by one of the spacer inserts when mounted on or in relation to the clamp.

6. A cutting assembly as claimed in claim 2, in which the clamp includes a mount and in which each of the connector inserts has a mount feature for engaging the mount to locate a selected connector insert on the clamp.

7. A cutting assembly as claimed in any one of claims 4 to 6, in which the clamp includes a mount and in which each of the spacer inserts has a mount feature for engaging the mount to locate a selected spacer insert on the clamp.

8. A cutting assembly as claimed in claim 6 or claim 7, in which the mount comprises a rod on which an insert having a channel formed in it can be mounted by sliding, preferably in which the mount includes a latch feature which can be engaged by an insert which has been slid fully along the rod so that further movement of the insert along the rod is inhibited.

9. A cutting assembly as claimed in claim 1, in which the connector arrangement comprises:
a. a plurality of connector inserts (30, 32, 34, 220, 222, 224) which can be mounted individually or in one or more combinations on or relative to the clamp, providing a physical connection between the reference and support surfaces which defines the inclination of the support surface relative to the reference surface so that the inclination of the support surface relative to the reference surface can be varied by changing one or more of the connector inserts, and
b. a plurality of spacer inserts (36, 38, 40) which can be mounted individually or in one or more combinations on or relative to the clamp, providing a physical connection between the reference and support surfaces so that the distance between the support surface and the reference surface can be varied by changing one or more of the spacer inserts,
and in which each of the connector inserts and each of the spacer inserts has a mateable connector feature for connecting a selected connector insert and a selected spacer insert to one another.

10. A cutting assembly as claimed claim 9, in which each of the support surface and the reference surface is approximately planar and which includes a first spacer insert and a second spacer insert, and in which the angle between the planes defined by the support and reference surfaces when the first spacer insert is connected to a selected connector insert is the same as the angle between the planes defined by the support and reference surfaces when the second spacer insert is connected to the selected connector insert.

11. A cutting assembly as claimed in any one of claims 1 to 10, in which the cutting head is a saw blade, preferably in which the clamp has a slot (18, 20) formed in it which the saw blade can move in with a reciprocating action, and the support surface is provided within the slot.

12. A cutting assembly as claimed in any one of claims 1 to 11, in which each of the support surface and the reference surface is approximately planar.

13. A cutting assembly as claimed in claim 12, in which the connector arrangement comprises a first connector insert and a second connector insert, in which, when the first connector insert is mounted on or in relation to the clamp, the plane defined by the reference surface is approximately parallel to the plane defined by the support surface, and in which, when the second connector insert is mounted on or in relation to the clamp, the angle between the planes defined by the support and reference surfaces is greater than 0°.

14. A cutting assembly as claimed in claim 12, which includes a neutral connector insert (220) which can be mounted on or in relation to the clamp, and in which, when the neutral connector insert is mounted on or in relation to the clamp, the plane defined by the reference surface is approximately parallel to the plane defined by the support surface.

15. A cutting assembly as claimed in claim 1, in which the connector assembly includes:
a. a primary insert (42) which can be mounted on or in relation to the clamp so that, when the primary insert is mounted on or in relation to the clamp, the plane defined by the reference surface is parallel to the plane defined by the support surface, and
b. a plurality of spacer inserts (36, 38, 40) which can be mounted individually or in one or more combinations on or relative to the clamp, providing a physical connection between the reference and support surfaces which defines the distance between the support surface and the reference surface so that the distance between the support surface and the reference surface can be varied by changing one or more of the spacer inserts,
in which the distance between the reference and support surfaces when the primary insert is mounted on the clamp is greater than the greatest of the distances between the reference and support surfaces that are available when any of the spacer inserts are mounted on the clamp.

## Patentansprüche

1. Schneidanordnung zum Resezieren der Kniescheibe (504) eines Patienten während einer Knieoperation, die Folgendes umfasst:
a. ein Schneidinstrument mit einem Schneidkopf zum Schneiden der Kniescheibe auf einer ausgewählten Resektionsebene,
b. eine Klemmvorrichtung (2) für eine Kniescheibe, mit gegenüberliegenden Backen (14, 16), die aufeinander zu bewegt werden können, um die Kniescheibe zwischen sich festzuklemmen, wobei die Klemmvorrichtung folgendes bereitstellt:
i. eine Referenzfläche (208) zum Eingriff mit der hinteren Oberfläche der Kniescheibe,
ii. eine Auflagefläche zum Abstützen des Schneidkopfes des Schneidinstruments, wodurch das Schneidinstrument relativ zur Klemmvorrichtung positioniert wird,
wobei die Anordnung eine Verbindungsanordnung einschließt, die eine physikalische Verbindung zwischen der Referenz- und der Auflagefläche herstellt, um die Neigung der Auflagefläche relativ zur Referenzfläche zu definieren,
**dadurch gekennzeichnet, dass** die Verbindungsanordnung so eingestellt werden kann, dass die Neigung der Auflagefläche gegenüber der Referenzfläche variiert werden kann.

2. Schneidanordnung nach Anspruch 1, bei der die Verbindungsanordnung eine Vielzahl von Verbindungseinsätzen (30, 32, 34, 220, 222, 224) umfasst, die einzeln oder in einer oder mehreren Kombinationen an oder relativ zu der Klemmvorrichtung montiert werden können und eine physikalische Verbindung zwischen der Referenz- und der Auflagefläche herstellen, die die Neigung der Auflagefläche relativ zur Referenzfläche definiert, und bei der eine Veränderung der Neigung der Auflagefläche relativ zur Referenzfläche das Auswechseln eines oder mehrerer der Verbindungseinsätze erfordert, die an oder relativ zu der Klemmvorrichtung montiert sind, wobei die Verbindungseinsätze vorzugsweise Markierungen (257) aufweisen, die die jeweiligen Neigungen der Auflagefläche relativ zur Referenzfläche anzeigen.

3. Schneidanordnung nach Anspruch 1 oder Anspruch 2, bei der die Verbindungsanordnung den Abstand zwischen der Auflagefläche und der Referenzfläche definiert und bei der der Abstand zwischen der Auflagefläche und der Referenzfläche durch Einstellen der Verbindungsanordnung variiert werden kann.

4. Schneidanordnung nach Anspruch 3, bei der die Verbindungsanordnung eine Vielzahl von Abstandseinsätzen (36, 38, 40) umfasst, die einzeln oder in einer oder mehreren Kombinationen an oder relativ zu der Klemmvorrichtung montiert werden können und eine physikalische Verbindung zwischen der Referenz- und der Auflagefläche herstellen, und bei der das Variieren des Abstands zwischen der Auflagefläche und der Referenzfläche das Auswechseln eines oder mehrerer der Abstandseinsätze erfordert, die an oder relativ zu der Klemmvorrichtung montiert sind, wobei die Abstandseinsätze vorzugsweise Markierungen (272) aufweisen, die die Abstände zwischen der Auflagefläche und der Referenzfläche anzeigen.

5. Schneidanordnung nach Anspruch 4, bei der die Auflagefläche und/oder die Referenzfläche durch einen der Abstandseinsätze bereitgestellt wird, wenn dieser an oder relativ zu der Klemmvorrichtung montiert ist.

6. Schneidanordnung nach Anspruch 2, bei der die Klemmvorrichtung eine Halterung einschließt, und bei der jeder der Verbindungseinsätze ein Halterungsmerkmal zum Eingreifen in die Halterung aufweist, um einen ausgewählten Verbindungseinsatz an der Klemmvorrichtung zu positionieren.

7. Schneidanordnung nach einem der Ansprüche 4 bis 6, bei der die Klemmvorrichtung eine Halterung einschließt und bei der jeder der Abstandseinsätze ein Halterungsmerkmal zum Eingreifen in die Halterung aufweist, um einen ausgewählten Abstandseinsatz an der Klemmvorrichtung zu positionieren.

8. Schneidanordnung nach Anspruch 6 oder Anspruch 7, bei der die Halterung eine Stange umfasst, auf der ein Einsatz mit einem darin ausgebildeten Kanal durch Aufschieben montiert werden kann, wobei die Halterung vorzugsweise ein Verriegelungsmerkmal aufweist, in die ein Einsatz eingreifen kann, der vollständig entlang der Stange geschoben wurde, sodass eine weitere Bewegung des Einsatzes entlang der Stange verhindert wird.

9. Schneidanordnung nach Anspruch 1, bei der die Verbindungsanordnung Folgendes umfasst:
a. eine Vielzahl von Verbindungseinsätzen (30, 32, 34, 220, 222, 224), die einzeln oder in einer oder mehreren Kombinationen an oder relativ zu der Klemmvorrichtung montiert werden können und eine physikalische Verbindung zwischen der Referenz- und der Auflagefläche herstellen, die die Neigung der Auflagefläche relativ zur Referenzfläche definiert, so dass die Neigung der Auflagefläche relativ zur Referenzfläche durch Auswechseln eines oder mehrerer Verbindungseinsätze variiert werden kann, und
b. eine Vielzahl von Abstandseinsätzen (36, 38, 40), die einzeln oder in einer oder mehreren Kombinationen an oder relativ zu der Klemmvorrichtung montiert werden können und eine physikalische Verbindung zwischen der Referenz- und der Auflagefläche herstellen, so dass der Abstand zwischen der Auflagefläche und der Referenzfläche durch Auswechseln eines oder mehrerer Abstandseinsätze variiert werden kann,
und wobei jeder der Verbindungseinsätze und jeder der Abstandseinsätze über ein Steckverbindungsmerkmal verfügt, um einen ausgewählten Verbindungseinsatz und einen ausgewählten Abstandseinsatz miteinander zu verbinden.

10. Schneidanordnung nach Anspruch 9, bei der sowohl die Auflagefläche als auch die Referenzfläche etwa eben sind und die einen ersten Abstandseinsatz und einen zweiten Abstandseinsatz einschließt, und bei der der Winkel zwischen den durch die Auflage- und Referenzflächen definierten Ebenen, wenn der erste Abstandseinsatz mit einem ausgewählten Verbindungseinsatz verbunden ist, der gleiche ist wie der Winkel zwischen den durch die Auflage- und Referenzflächen definierten Ebenen, wenn der zweite Abstandseinsatz mit dem ausgewählten Verbindungseinsatz verbunden ist.

11. Schneidanordnung nach einem der Ansprüche 1 bis 10, bei der der Schneidkopf ein Sägeblatt ist, wobei die Klemmvorrichtung vorzugsweise einen darin ausgebildeten Schlitz (18, 20) aufweist, in dem sich das Sägeblatt hin- und herbewegen kann, und die Auflagefläche innerhalb des Schlitzes vorgesehen ist.

12. Schneidanordnung nach einem der Ansprüche 1 bis 11, bei der sowohl die Auflagefläche als auch die Referenzfläche etwa eben sind.

13. Schneidanordnung nach Anspruch 12, bei der die Verbindungsanordnung einen ersten Verbindungseinsatz und einen zweiten Verbindungseinsatz umfasst, wobei, wenn der erste Verbindungseinsatz an oder relativ zu der Klemmvorrichtung montiert ist, die durch die Referenzfläche definierte Ebene etwa parallel zu der durch die Auflagefläche definierten Ebene ist, und bei der, wenn der zweite Verbindungseinsatz an oder relativ zu der Klemmvorrichtung montiert ist, der Winkel zwischen den durch die Auflage- und Referenzflächen definierten Ebenen größer als 0° ist.

14. Schneidanordnung nach Anspruch 12, die einen neutralen Verbindungseinsatz (220) umfasst, der an oder relativ zu der Klemmvorrichtung montiert werden kann, und wobei, wenn der neutrale Verbindungseinsatz an oder relativ zu der Klemmvorrichtung montiert ist, die durch die Referenzfläche definierte Ebene etwa parallel zu der durch die Auflagefläche definierten Ebene ist.

15. Schneidanordnung nach Anspruch 1, bei der die Verbindungsanordnung Folgendes umfasst:
a. einen Primäreinsatz (42), der an oder relativ zu der Klemmvorrichtung montiert werden kann, so dass, wenn der Primäreinsatz an oder relativ zu der Klemmvorrichtung montiert ist, die durch die Referenzfläche definierte Ebene parallel zu der durch die Auflagefläche definierten Ebene ist, und
b. eine Vielzahl von Abstandseinsätzen (36, 38, 40), die einzeln oder in einer oder mehreren Kombinationen an oder relativ zu der Klemmvorrichtung montiert werden können und eine physikalische Verbindung zwischen der Referenz- und der Auflagefläche herstellen, die den Abstand zwischen der Auflagefläche und der Referenzfläche definiert, so dass der Abstand zwischen der Auflagefläche und der Referenzfläche durch Auswechseln eines oder mehrerer Abstandseinsätze variiert werden kann,
wobei der Abstand zwischen der Referenz- und der Auflagefläche, wenn der Primäreinsatz an der Klemmvorrichtung montiert ist, größer ist als der größte der Abstände zwischen der Referenz- und der Auflagefläche, die verfügbar sind, wenn einer der Abstandseinsätze an der Klemmvorrichtung montiert ist.

## Revendications

1. Ensemble de coupe de résection d'une rotule (504) d'un patient lors d'une opération du genou, comprenant :
a. un instrument de coupe ayant une tête de coupe pour couper la rotule sur un plan de résection sélectionné,
b. une pince (2) pour une rotule ayant des mâchoires opposées (14, 16) qui peuvent être déplacées l'une vers l'autre pour serrer la rotule entre elles, la pince fournissant :
i. une surface de référence (208) pour entrer en prise avec la surface postérieure de la rotule,
ii. une surface de support pour supporter la tête de coupe de l'instrument de coupe, ce qui permet de positionner l'instrument de coupe par rapport à la pince,
dans lequel l'ensemble comporte un agencement d'élément de liaison qui assure une liaison physique entre les surfaces de référence et de support pour définir l'inclinaison de la surface de support par rapport à la surface de référence,
**caractérisé en ce que** l'agencement d'élément de liaison peut être ajusté pour faire varier l'inclinaison de la surface de support par rapport à la surface de référence.

2. Ensemble de coupe selon la revendication 1, dans lequel l'agencement d'élément de liaison comprend une pluralité d'inserts d'élément de liaison (30, 32, 34, 220, 222, 224) qui peuvent être montés individuellement ou dans une ou plusieurs combinaisons sur la pince ou par rapport à celle-ci, fournissant une liaison physique entre les surfaces de référence et de support qui définit l'inclinaison de la surface de support par rapport à la surface de référence, et dans lequel une variation de l'inclinaison de la surface de support par rapport à la surface de référence implique le changement d'un ou plusieurs des inserts d'élément de liaison qui sont montés sur la pince ou par rapport à celle-ci, de préférence dans lequel les inserts d'élément de liaison ont des marquages (257) sur eux qui indiquent des inclinaisons respectives de la surface de support par rapport à la surface de référence.

3. Ensemble de coupe selon la revendication 1 ou la revendication 2, dans lequel l'agencement d'élément de liaison définit la distance entre la surface d'appui et la surface de référence, et dans lequel la distance entre la surface de support et la surface de référence peut être variée en ajustant l'agencement d'élément de liaison.

4. Ensemble de coupe selon la revendication 3, dans lequel l'agencement d'élément de liaison comprend une pluralité d'inserts d'espacement (36, 38, 40) qui peuvent être montés individuellement ou dans une ou plusieurs combinaisons sur la pince ou par rapport à celle-ci, fournissant une liaison physique entre les surfaces de référence et de support, et dans lequel la variation de la distance entre la surface de support et la surface de référence implique le changement d'un ou plusieurs des inserts d'espacement qui sont montés sur la pince ou par rapport à celle-ci, de préférence dans lequel les inserts d'espacement ont des marquages (272) sur eux qui indiquent des distances entre la surface de support et la surface de référence.

5. Ensemble de coupe selon la revendication 4, dans lequel au moins l'une parmi la surface de support et la surface de référence est fournie par l'un des inserts d'espacement lorsqu'il est monté sur la pince ou en relation avec celle-ci.

6. Ensemble de coupe selon la revendication 2, dans lequel la pince comporte une monture et dans lequel chacun des inserts d'élément de liaison a une caractéristique de monture pour entrer en prise avec la monture afin de localiser un insert d'élément de liaison sélectionné sur la pince.

7. Ensemble de coupe selon l'une quelconque des revendications 4 à 6, dans lequel la pince comporte une monture et dans lequel chacun des inserts d'espacement a une caractéristique de monture pour entrer en prise avec la monture afin de localiser un insert d'espacement sélectionné sur la pince.

8. Ensemble de coupe selon la revendication 6 ou la revendication 7, dans lequel la monture comprend une tige sur laquelle un insert ayant un canal formé dans celui-ci peut être monté par glissement, de préférence dans lequel la monture comporte une caractéristique de verrouillage qui peut être mise en prise par un insert qui a été glissé complètement le long de la tige de sorte qu'un mouvement ultérieur de l'insert le long de la tige est inhibé.

9. Ensemble de coupe selon la revendication 1, dans lequel l'agencement d'élément de liaison comprend :
a. une pluralité d'inserts d'élément de liaison (30, 32, 34, 220, 222, 224) qui peuvent être montés individuellement ou dans une ou plusieurs combinaisons sur la pince ou par rapport à celle-ci, fournissant une liaison physique entre les surfaces de référence et de support qui définit l'inclinaison de la surface de support par rapport à la surface de référence de sorte que l'inclinaison de la surface de support par rapport à la surface de référence peut être variée en changeant un ou plusieurs des inserts d'élément de liaison, et
b. une pluralité d'inserts d'espacement (36, 38, 40) qui peuvent être montés individuellement ou dans une ou plusieurs combinaisons sur la pince ou par rapport à celle-ci, fournissant une liaison physique entre les surfaces de référence et de support de sorte que la distance entre la surface de support et la surface de référence peut être variée en changeant un ou plusieurs des inserts d'espacement,
et dans lequel chacun des inserts d'élément de liaison et chacun des inserts d'espacement a une caractéristique d'élément de liaison appariable pour la liaison d'un insert d'élément de liaison sélectionné et d'un insert d'espacement sélectionné l'un à l'autre.

10. Ensemble de coupe selon la revendication 9, dans lequel chacune de la surface de support et de la surface de référence sont approximativement planes et qui comporte un premier insert d'espacement et un second insert d'espacement, et dans lequel l'angle entre les plans définis par les surfaces de support et de référence lorsque le premier insert d'espacement est relié à un insert d'élément de liaison sélectionné est le même que l'angle entre les plans définis par les surfaces de support et de référence lorsque le second insert d'espacement est relié à l'insert d'élément de liaison sélectionné.

11. Ensemble de coupe selon l'une quelconque des revendications 1 à 10, dans lequel la tête de coupe est une lame de scie, de préférence dans lequel la pince a une fente (18, 20) formée dans celle-ci dans laquelle la lame de scie peut se déplacer dans un mouvement de va-et-vient, et la surface d'appui est prévue à l'intérieur de la fente.

12. Ensemble de coupe selon l'une quelconque des revendications 1 à 11, dans lequel chacune de la surface de support et de la surface de référence est approximativement plane.

13. Ensemble de coupe selon la revendication 12, dans lequel l'agencement d'élément de liaison comprend un premier insert d'élément de liaison et un second insert d'élément de liaison, dans lequel, lorsque le premier insert d'élément de liaison est monté sur la pince ou en relation avec celle-ci, le plan défini par la surface de référence est approximativement parallèle au plan défini par la surface de support, et dans lequel, lorsque le second insert d'élément de liaison est monté sur la pince ou en relation avec celle-ci, l'angle entre les plans définis par les surfaces d'appui et de référence est supérieur à 0°.

14. Ensemble de coupe selon la revendication 12, qui comporte un insert d'élément de liaison (220) neutre qui peut être monté sur la pince ou en relation avec celle-ci, et dans lequel, lorsque l'insert d'élément de liaison neutre est monté sur la pince ou en relation avec celle-ci, le plan défini par la surface de référence est approximativement parallèle au plan défini par la surface de support.

15. Ensemble de coupe selon la revendication 1, dans lequel l'ensemble d'élément de liaison comporte :
a. un insert primaire (42) qui peut être monté sur la pince ou en relation avec celle-ci de sorte que, lorsque l'insert primaire est monté sur la pince ou en relation avec celle-ci, le plan défini par la surface de référence est parallèle au plan défini par la surface de support, et
b. une pluralité d'inserts d'espacement (36, 38, 40) qui peuvent être montés individuellement ou dans une ou plusieurs combinaisons sur la pince ou par rapport à celle-ci, fournissant une liaison physique entre les surfaces de référence et de support qui définit la distance entre la surface de support et la surface de référence de sorte que la distance entre la surface de support et la surface de référence peut être variée en changeant un ou plusieurs des inserts d'espacement,
dans lequel la distance entre les surfaces de référence et de support lorsque l'insert primaire est monté sur la pince est supérieure à la plus grande des distances entre les surfaces de référence et de support qui sont disponibles lorsque l'un quelconque des inserts d'espacement est monté sur la pince.
